(19) Europäisches Patentamt
European Patent Office
Office européen des brevets

(11) **EP 4 624 896 A1**

(12) **EUROPEAN PATENT APPLICATION**
published in accordance with Art. 153(4) EPC

(43) Date of publication:
01.10.2025 Bulletin 2025/40

(21) Application number: 23894275.9

(22) Date of filing: 06.10.2023

(51) International Patent Classification (IPC):
$G01N\ 15/1429^{(2024.01)}$    $G01N\ 37/00^{(2006.01)}$
$G01N\ 21/05^{(2006.01)}$    $G01N\ 21/64^{(2006.01)}$
$G01N\ 15/14^{(2024.01)}$

(52) Cooperative Patent Classification (CPC):
G01N 15/14; G01N 15/1429; G01N 21/05;
G01N 21/64; G01N 37/00

(86) International application number:
PCT/JP2023/036503

(87) International publication number:
WO 2024/111263 (30.05.2024 Gazette 2024/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 24.11.2022 JP 2022187462

(71) Applicant: **Sony Group Corporation**
**Tokyo 108-0075 (JP)**

(72) Inventor: **TAHARA Katsutoshi**
**Tokyo 108-0075 (JP)**

(74) Representative: **D Young & Co LLP**
**3 Noble Street**
**London EC2V 7BQ (GB)**

(54) **BIOLOGICAL SAMPLE ANALYSIS DEVICE, BIOLOGICAL SAMPLE ANALYSIS SYSTEM, AND METHOD FOR VERIFYING STATUS OF BIOLOGICAL SAMPLE ANALYSIS DEVICE**

(57)    The present disclosure aims to provide a new technique for verifying a status of a device.

The present disclosure provides a biological sample analyzer including an information processing unit configured to perform information processing using signal intensity data on light generated by irradiating a flow channel through which particles are flowing with light. The information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying the status of the device.

*FIG. 3*

| | |
|---|---|
| DATA SET ACQUISITION STEP | S11 |
| TRIGGER SLICE SETTING STEP | S12 |
| BACKGROUND DATA ACQUISITION STEP | S13 |
| DATA SEPARATION STEP | S14 |
| DATA REPLACEMENT STEP | S15 |
| EVALUATION VALUE CALCULATION STEP | S16 |

EP 4 624 896 A1

**Description**

TECHNICAL FIELD

The present disclosure relates to a biological sample analyzer, a biological sample analysis system, and a method for verifying the status of the biological sample analyzer. More specifically, the present disclosure relates to a biological sample analyzer and a biological sample analysis system that perform analysis on the basis of light generated by irradiating particles flowing through a flow channel with light, and a method for verifying the status of the biological sample analyzer.

BACKGROUND ART

For example, a particle population such as cells, microorganisms, and liposomes is labeled with a fluorescent dye, and the intensity and/or pattern of fluorescence generated from the fluorescent dye excited by irradiating each particle of the particle population with laser light is measured, thereby measuring the characteristics of the particles. Examples of a particle analyzer that performs the measurement include a flow cytometer and a cell sorter.

The flow cytometer and the cell sorter may be configured to irradiate particles flowing in a line through a flow channel with laser light (excitation light) having a specific wavelength and detect fluorescence and/or scattered light emitted from each particle to analyze a plurality of particles one by one. Such devices can convert light detected by a photodetector into an electrical signal, quantify the electrical signal, and perform statistical analysis to determine characteristics, for example, the type, size, structure, and the like of each particle.

Quality control (QC) is important for such a particle analyzer from the viewpoint of maintaining data quality. For example, regarding quality control, Patent Document 1 below discloses an information processing device including an information processing unit that acquires a plurality of fluorescence intensities on the basis of fluorescence signals from a sample containing a plurality of particles labeled with fluorescent dyes having different fluorescence intensities, recognizes an intensity range for each of the plurality of fluorescence intensities detected on the basis of a fluorescence intensity ratio of the sample, and calculates information regarding sensitivity of a fluorescence detection unit.

CITATION LIST

PATENT DOCUMENT

Patent Document 1: WO 2016/185755 A

SUMMARY OF THE INVENTION

PROBLEMS TO BE SOLVED BY THE INVENTION

To verify the status of a biological sample analyzer such as a flow cytometer or a cell sorting system, a device status verification sample such as eight-peak beads is flowed into a flow channel, and light detection processing is performed. For example, evaluation values such as linearity, molecules of equivalent soluble fluorochrome (MESF), a Q value, and a B value are calculated on the basis of the data obtained as a result of the light detection processing.

The eight-peak beads include seven-level fluorescent beads and one type of non-fluorescent beads. In general, MESF is an evaluation value based on the mean fluorescence intensity (MFI) of Dim1, and the components that make up the data primarily include the fluorescence components of the non-fluorescent beads and the background of the device.

The background of the device is directly related to its performance, so it is considered necessary to verify this value. Here, the non-fluorescent beads produces fluorescence, although the produced fluorescence is significantly weaker than the fluorescence generated by the fluorescent beads. In a case where the non-fluorescent beads is high in fluorescence level, it may be difficult to accurately evaluate the performance of the background of the device. Furthermore, when processing such as sterilization is performed on the verification sample such as eight-peak beads, the fluorescence level of the non-fluorescent beads can vary, which may affect the performance evaluation of the background of the device.

It is therefore an object of the present disclosure to provide a new technique for verifying a device status. In particular, the object is to provide a technique for properly verifying the device status not only with a standard verification sample but also with a verification sample whose fluorescence level has varied as described above.

SOLUTIONS TO PROBLEMS

The present disclosure provides

a biological sample analyzer including:

an information processing unit configured to perform information processing using signal intensity data on light generated by irradiating a flow channel through which particles are flowing with light, in which the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

The information processing unit may calculate one or more evaluation values for verifying the status of the device using a data set obtained after the data replacement processing.

The one or more evaluation values may include at least any one of a precision evaluation value of the device, a linearity evaluation value of the device, or a sensitivity evaluation value of the device.

Signal intensity data of a particle group with the lowest signal intensity among the plurality of types of particle groups may be replaced in the data replacement step.

The sample containing the plurality of types of particle groups may contain one type of non-fluorescent particle group and one or more types of fluorescent particle groups, and

signal intensity data of the one type of non-fluorescent particle group may be replaced in the data replacement step.

The biological sample analyzer may be configured to perform a trigger slice setting step of setting a trigger slice using the data set.

The biological sample analyzer may be configured to acquire the signal intensity data on light generated by irradiating the flow channel through which no particles are flowing with light using the trigger slice.

The biological sample analyzer may be configured to perform a background data acquisition step of acquiring the signal intensity data on light generated by irradiating the flow channel through which no particles are flowing with light.

The acquisition of the signal intensity data in the background data acquisition step may be performed in a state where the sample is not flowed into the flow channel, but only a sheath liquid is flowed into the flow channel.

The acquisition of the signal intensity data in the background data acquisition step may be performed at predetermined time intervals.

The number of pieces of the signal intensity data acquired in the background data acquisition step may be identical to the number set on the basis of the number of pieces of event data in the data set and the number of peaks formed by the verification sample.

The number of pieces of the signal intensity data acquired in the background data acquisition step may be identical to the number set on the basis of the number of pieces of the signal intensity data replaced in the data replacement step.

The biological sample analyzer may be configured to further perform a data separation step of separating the data set into data corresponding to each of the plurality of types of particle groups.

The biological sample analyzer may be configured to perform the data separation through clustering processing in the data separation step.

The biological sample analyzer may be configured to replace, in the data replacement step, signal intensity data of a particle group with the lowest fluorescence level among the plurality of types of particle groups with the signal intensity data acquired by irradiating the flow channel through which no particles are flowing with light.

The sample containing the plurality of types of particle groups may contain one type of non-fluorescent particle group and one or more types of fluorescent particle groups, and

the biological sample analyzer may be configured to replace, in the data replacement step, signal intensity data of the one type of non-fluorescent particle group with the signal intensity data acquired by irradiating the flow channel through which no particles are flowing with light.

The biological sample analyzer may serve as a flow cytometer.

The present disclosure further provides

a biological sample analysis system including:

an information processing unit configured to perform information processing using signal intensity data on light generated by irradiating a flow channel through which particles are flowing with light, in which the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

The present disclosure further provides a method for verifying a status of a biological sample analyzer, the method including:
a data replacement step of causing a sample containing a plurality of types of particle groups having varying fluorescence intensity levels to flow into a flow channel, and replacing signal intensity data on light generated by irradiating, with light, on any one type of particle group in a data set of signal intensity data on light generated by irradiating, with light, particles flowing through the flow channel with signal intensity data acquired by irradiating the flow channel through which no particles are flowing with light.

BRIEF DESCRIPTION OF DRAWINGS

Fig. 1 is an example of plot data for describing fluctuations in fluorescence level.
Fig. 2 is a diagram illustrating a configuration example of a biological sample analyzer of the present disclosure.
Fig. 3 is an example of a flowchart of verification processing.
Fig. 4 is a diagram for describing a trigger slice.
Fig. 5 is a diagram for describing differences in data separation results using different fluorescent dye channels.
Fig. 6 is a diagram for describing clamping.
Fig. 7 is a table showing MFI and MEF.
Fig. 8 is an example of plot data for describing fluctuations in fluorescence level.
Fig. 9 is a table showing differences and difference rates of evaluation values.
Fig. 10 is a diagram illustrating a configuration example of the biological sample analyzer of the present disclosure.
Fig. 11 is a diagram illustrating an example of a flowchart of biological particle sorting processing.
Fig. 12 is a schematic enlarged view of a particle sorting unit.
Fig. 13 is a diagram illustrating a schematic configuration example of a control unit.

MODE FOR CARRYING OUT THE INVENTION

Hereinafter, preferred modes for carrying out the present disclosure will be described. Note that embodiments described below illustrate representative embodiments of the present disclosure, and the scope of the present disclosure is not limited only to these embodiments. Note that the present disclosure will be described in the following order.

1. First embodiment (biological sample analyzer)

(1) Basic concept
(2) Configuration example
(3) Verification processing
(4) Examples
(5) Configuration example of biological particle sorting device

2. Second embodiment (biological sample analysis system)
3. Third embodiment (verification method of biological sample analyzer and program for executing verification method)

1. First embodiment (biological sample analyzer)

(1) Basic concept

Examples of a sample (hereinafter, also referred to as "device status verification sample" or "verification sample") used for verifying the device status of a biological sample analyzer include a sample containing a plurality of types of particle groups having varying fluorescence intensity levels. The eight-peak beads, which are an example of the sample, contains seven types of fluorescent beads having varying fluorescence levels and one type of non-fluorescent bead as described above.

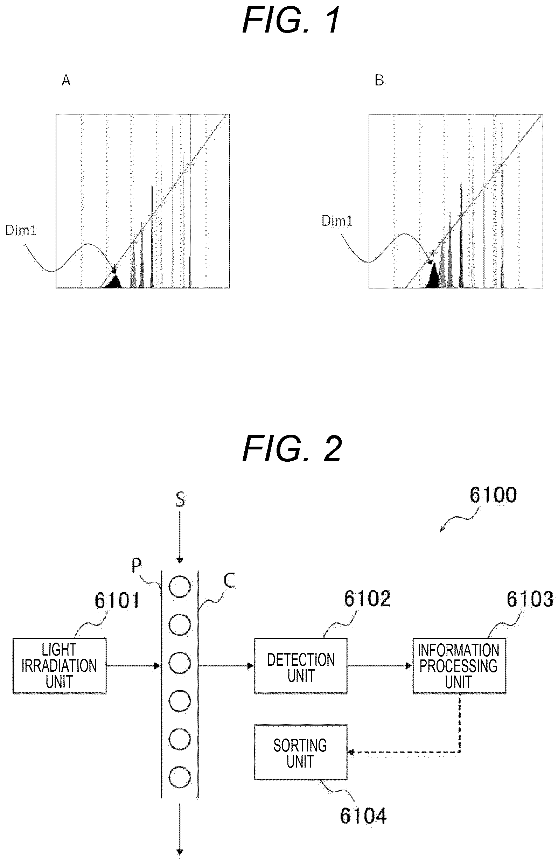

As described above, when processing such as sterilization is performed on the verification sample such as eight-peak beads, the fluorescence levels of the beads (such as non-fluorescent beads) may change. An example of how the fluorescence levels change will be described with reference to Fig. 1. A of the drawing shows plot data of signal intensity obtained by performing flow cytometry on unsterilized eight-peak beads. B of the drawing shows plot data of signal intensity obtained by performing flow cytometry on sterilized eight-peak beads. The horizontal axis of such plot data represents the signal intensity, and the vertical axis represents the number of events.

A of the drawing shows that the eight peaks are separated from each other. On the other hand, B of the drawing shows that the fluorescence levels of particle groups with lower fluorescence intensity levels fluctuate; particularly, the fluorescence intensity level of a particle group Dim1 with the lowest fluorescence intensity fluctuates significantly, and is increasing. Such fluctuations in fluorescence intensity levels adversely affects, for example, an evaluation value (such as MESF) for evaluating the status of a device. The particle group Dim1 in the drawings includes non-fluorescent beads, and even the non-fluorescent beads may experience fluctuations in fluorescence intensity level due to sterilization.

The biological sample analyzer according to the present disclosure performs verification processing of verifying its device status using the verification sample. In the verification processing, the signal intensity data on one type of particle group among the plurality of types of particle groups, the signal intensity data belonging to a data set obtained as a result of light detection processing performed on the sample, is replaced with the signal intensity data on the background. For example, in the verification processing using the eight-peak beads, for example, the signal intensity data on the non-fluorescent beads in the data set obtained as a result of the light detection processing performed on the eight-peak beads is replaced with the signal intensity data on the background (signal intensity data obtained by irradiating the flow channel through which no particles are flowing with light).

Using the data set obtained as a result of the replacement, the evaluation value of the device status becomes a value that reflects the background. It is therefore possible to properly evaluate, for example, even in a case where the beads are sterilized, the device status without being affected by changes in the status of the beads.

Furthermore, the replacement can also be applied to verification processing in which unsterilized beads are used. That is, according to the present disclosure, the device status can be properly evaluated in both a case where unsterilized beads are used and a case where sterilized beads are used.

In the present disclosure, for example, to use beads with fluorescence levels that have fluctuated due to sterilization or the like, data based only on the background may be applied to Dim1. Accordingly, the evaluation value of the device status such as MESF reflects the background to represent the device's performance, and is not affected by the status of the beads. In addition, this technique is applicable to eight-peak beads that has not fluctuated in fluorescence levels.

When the fluorescence level of the non-fluorescent beads is high, it may not be possible to properly evaluate the device status. In this case, the MESF does not accurately represent the device status. In particular, an increase in the level of the non-fluorescent beads due to sterilization or the like tends to make the evaluation difficult. This is one of the reasons why the evaluation of the device status using sterilized beads has not been performed on analyzers such as flow cytometers.

In one embodiment, the biological sample analyzer according to the present disclosure may include an information processing unit configured to perform information processing using signal intensity data on light generated by irradiating a flow channel through which particles are flowing with light, in which the information processing unit may be configured to perform a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device. The data set subjected to the replacement, obtained as a result of the data replacement step, is useful for properly verifying the device status.

The biological sample analyzer may include, for example, a light irradiation unit that irradiates a flow channel through which particles are flowing with light, a detection unit that detects light generated by the light irradiation, and an information processing unit that controls the light irradiation unit and the detection unit.

Hereinafter, first, a configuration example of the biological sample analyzer according to the present disclosure will be described, and next, verification processing performed by the device will be described.

(2) Configuration example

Fig. 2 illustrates a configuration example of the biological sample analyzer of the present disclosure. A biological sample analyzer 6100 illustrated in Fig. 2 includes a light irradiation unit 6101 that irradiates a biological sample S flowing through a flow channel C with light, a detection unit 6102 that detects light generated by irradiating the biological sample S with light, and an information processing unit 6103 that processes information regarding the light detected by the detection unit. The biological sample analyzer 6100 is a flow cytometer or an imaging cytometer, for example. The biological sample analyzer 6100 may include a sorting unit 6104 that sorts out specific biological particles P in a biological sample. The biological sample analyzer 6100 including the sorting unit is a cell sorter, for example.

(Biological Sample)

The biological sample S may be a liquid sample containing biological particles. The biological particles are cells or non-cellular biological particles, for example. The cells may be living cells, and more specific examples thereof include blood cells such as erythrocytes and leukocytes, and germ cells such as sperms and fertilized eggs. Also, the cells may be those directly collected from a sample such as whole blood, or may be cultured cells obtained after culturing. The non-cellular biological particles are extracellular vesicles, or particularly, exosomes and microvesicles, for example. The biological particles may be labeled with one or more labeling substances (such as a dye (particularly, a fluorescent dye) and a fluorochrome-labeled antibody). Note that particles other than biological particles may be analyzed by the biological sample analyzer of the present disclosure, and beads or the like may be analyzed for calibration or the like.

(Flow Channel)

The flow channel C is designed so that a flow of the biological sample S is formed. In particular, the flow channel C may be designed so that a flow in which the biological particles contained in the biological sample are aligned substantially in one row is formed. The flow channel structure including the flow channel C may be designed so that a laminar flow is formed. In particular, the flow channel structure is designed so that a laminar flow in which the flow of the biological sample (a sample flow) is surrounded by the flow of a sheath liquid is formed. The design of the flow channel structure may be appropriately selected by a person skilled in the art, or a known one may be adopted. The flow channel C may be formed in a flow channel structure such as a microchip (a chip having a flow channel on the order of micrometers) or a flow cell. The width of the flow channel C is 1 mm or smaller, or particularly, may not be smaller than 10 $\mu$m and not greater than 1 mm. The flow channel C and the flow channel structure including the flow channel C may be formed by a material such as plastic or glass.

The biological sample analyzer of the present disclosure is designed so that the biological sample flowing in the flow channel C, or particularly, the biological particles in the biological sample are irradiated with light from the light irradiation unit 6101. The biological sample analyzer of the present disclosure may be designed so that the irradiation point (interrogation point) of light on the biological sample is located in the flow channel structure in which the flow channel C is formed, or may be designed so that the irradiation point is located outside the flow channel structure. An example of the former case may be a configuration in which the light is emitted onto the flow channel C in a microchip or a flow cell. An example of the latter case may be a configuration in which the biological particles after exiting the flow channel structure (particularly, the nozzle portion thereof) are irradiated with the light, such as a jet-in-air flow cytometer.

(Light Irradiation Unit)

The light irradiation unit 6101 includes a light source unit that emits light, and a light guide optical system that guides the light to the irradiation point. The light source unit includes one or more light sources. The type of the light source(s) is a laser light source or an LED, for example. The wavelength of light to be emitted from each light source may be any wavelength of ultraviolet light, visible light, and infrared light. The light guide optical system includes optical components such as beam splitters, mirrors, or optical fibers, for example. The light guide optical system may also include a lens group for condensing light, and includes an objective lens, for example. There may be one or more irradiation points at which the biological sample and light intersect. The light irradiation unit 6101 may be designed to collect light emitted onto one irradiation point from one light source or different light sources.

(Detection Unit)

The detection unit 6102 includes at least one photodetector that detects light generated by emitting light onto biological particles. The light to be detected may be fluorescence or scattered light (such as one or more of the following: forward scattered light, backscattered light, and side scattered light), for example. Each photodetector includes one or more light receiving elements, and has a light receiving element array, for example. Each photodetector may include one or more photomultiplier tubes (PMTs) and/or photodiodes such as APDs and MPPCs, as the light receiving elements. The photodetector includes a PMT array in which a plurality of PMTs is arranged in a one-dimensional direction, for example. The detection unit 6102 may also include an image sensor such as a CCD or a CMOS. With the image sensor, the detection unit 6102 can acquire an image (such as a bright-field image, a dark-field image, or a fluorescent image, for example) of biological particles.

The detection unit 6102 includes a detection optical system that causes light of a predetermined detection wavelength to reach the corresponding photodetector. The detection optical system includes a spectroscopic unit such as a prism or a diffraction grating, or a wavelength separation unit such as a dichroic mirror or an optical filter. The detection optical system is designed to disperse the light generated by light irradiation to biological particles, for example, and detect the dispersed light with a larger number of photodetectors than the number of fluorescent dyes with which the biological particles are labeled. A flow cytometer including such a detection optical system is called a spectral flow cytometer. Further, the detection optical system is designed to separate the light corresponding to the fluorescence wavelength band of a specific fluorescent dye from the light generated by the light irradiation to the biological particles, for example, and cause the corresponding photodetector to detect the separated light.

The detection unit 6102 may also include a signal processing unit that converts an electrical signal obtained by a photodetector into a digital signal. The signal processing unit may include an A/D converter as a device that performs the conversion. The digital signal obtained by the conversion performed by the signal processing unit can be transmitted to the information processing unit 6103. The digital signal can be handled as data related to light (hereinafter, also referred to as "light data") by the information processing unit 6103. The light data may be light data including fluorescence data, for example. More specifically, the light data may be data of light intensity, and the light intensity may be light intensity data of light including fluorescence (the light intensity data may include feature quantities such as area, height, and width).

(Information Processing Unit)

The information processing unit 6103 includes a processing unit that performs processing of various kinds of data (light data, for example), and a storage unit that stores various kinds of data, for example. In a case where the processing unit acquires the light data corresponding to a fluorescent dye from the detection unit 6102, the processing unit can perform fluorescence leakage correction (a compensation process) on the light intensity data. In the case of a spectral flow cytometer, the processing unit also performs a fluorescence separation process on the light data, and acquires the light intensity data corresponding to the fluorescent dye. The fluorescence separation process may be performed by an unmixing method disclosed in JP 2011-232259 A, for example. In a case where the detection unit 6102 includes an image sensor, the processing unit may acquire morphological information about the biological particles, on the basis of an image acquired by the image sensor. The storage unit may be designed to be capable of storing the acquired light data. The storage unit may be designed to be capable of further storing spectral reference data to be used in the unmixing process.

In a case where the biological sample analyzer 6100 includes the sorting unit 6104 described later, the information processing unit 6103 can determine whether to sort the biological particles, on the basis of the light data and/or the morphological information. The information processing unit 6103 then controls the sorting unit 6104 on the basis of the result of the determination, and the biological particles can be sorted by the sorting unit 6104.

The information processing unit 6103 may be designed to be capable of outputting various kinds of data (such as light data and images, for example). For example, the information processing unit 6103 can output various kinds of data (such as a two-dimensional plot or a spectrum plot, for example) generated on the basis of the light data. The information processing unit 6103 may also be designed to be capable of accepting inputs of various kinds of data, and accepts a gating process on a plot by a user, for example. The information processing unit 6103 may include an output unit (such as a display, for example) or an input unit (such as a keyboard, for example) for performing the output or the input.

The information processing unit 6103 may be designed as a general-purpose computer, and may be designed as an information processing device that includes a CPU, a RAM, and a ROM, for example. The information processing unit 6103 may be included in the housing in which the light irradiation unit 6101 and the detection unit 6102 are included, or may be located outside the housing. Further, the various processes or functions to be executed by the information processing unit 6103 may be realized by a server computer or a cloud connected via a network.

(Sorting Unit)

The sorting unit 6104 performs sorting of biological particles, in accordance with the result of determination performed by the information processing unit 6103. The sorting method may be a method by which droplets containing biological particles are generated by vibration, electric charges are applied to the droplets to be sorted, and the traveling direction of the droplets is controlled by an electrode. The sorting method may be a method for sorting by controlling the traveling direction of biological particles in the flow channel structure. The flow channel structure has a control mechanism based on pressure (injection or suction) or electric charge, for example. An example of the flow channel structure may be a chip (the chip disclosed in JP 2020-76736 A, for example) that has a flow channel structure in which the flow channel C branches into a recovery flow channel and a waste liquid flow channel on the downstream side, and specific biological particles are collected in the recovery flow channel.

(3) Verification processing

(3-1) Overview of verification processing

The biological sample analyzer according to the present disclosure may be configured to perform analysis processing on a biological sample on the basis of light generated by irradiating particles flowing through a flow channel with light. For example, the biological sample analyzer may perform the verification processing of verifying the device status before or during the analysis processing. Note that the biological sample analyzer may perform the verification processing after the analysis processing.

In the verification processing, for example, the device status verification sample is flowed into the flow channel. Then, each particle contained in the verification sample is irradiated with light, and an evaluation value for evaluating the device status is calculated on the basis of the light generated by the light irradiation. The verification sample may be a sample containing a plurality of types of particle groups having varying fluorescence intensity levels, and may be, for example, a sample that allows a plurality of peaks to be observed through the fluorescence separation process. The number of the peaks is, for example, 2 to 10, and particularly 3 to 10. Examples of the verification sample may include a bead population with a plurality of peaks, and more specifically, may include three-peak beads, four-peak beads, five-peak beads, six-peak beads, seven-peak beads, eight-peak beads, nine-peak beads, and ten-peak beads.

Any one of the plurality of types of particle groups contained in the verification sample may contain non-fluorescent particles. That is, the plurality of types of particle groups may contain one type of non-fluorescent particle group and one or more types of fluorescent particle groups. For example, the eight-peak beads contain one type of non-fluorescent bead and seven types of fluorescent beads. The present disclosure is particularly useful for performing verification processing using a verification sample containing a non-fluorescent particle group.

In the verification processing, a data set of signal intensity data on light generated by irradiating, with light, a sample containing a plurality of types of particle groups having varying fluorescence intensity levels is acquired.

Then, signal intensity data on light generated by irradiating any one type of particle group with light in the data set is replaced with signal intensity data acquired by irradiating the flow channel through which no particles are flowing with light.

The data set subjected to the replacement is useful for evaluating the status of the biological sample analyzer. For example, an evaluation value of at least any one of the precision of the device, the linearity of the device, or the sensitivity of the device may be calculated using the data set subjected to the replacement. The evaluation value calculated using the data set subjected to the replacement is useful for properly evaluating the device status, and particularly useful from the viewpoint of quality control (QC).

Note that, herein, a data set not subjected to the replacement is also referred to as "pre-replacement data set".

The data set subjected to the replacement is also referred to as "post-replacement data set".

Furthermore, the signal intensity data acquired by irradiating the flow channel through which no particles are flowing with light and used for the replacement is also referred to as "background data".

That is, in the present disclosure, the pre-replacement data set and the background data are acquired, and the post-replacement data set is generated from these pieces of data. Then, the evaluation value for evaluating the device status is calculated using the post-replacement data set. The evaluation value calculated as described above is useful for properly evaluating the device status even in a case where the verification sample is sterilized. It goes without saying that this verification processing is also applicable to a case where the verification sample is not sterilized.

In a case where the verification sample contains one type of non-fluorescent particle group and one or more types of fluorescent particle groups, the pre-replacement data set includes signal intensity data of the one type of non-fluorescent particle group and signal intensity data of each of the one or more types of fluorescent particle groups.

In this case, it is preferable that the signal intensity data of the non-fluorescent particle group in the pre-replacement data set be replaced with the background data. That is, through the replacement, the post-replacement data set including the background data and the signal intensity data of the one or more types of fluorescent particle groups is generated. The post-replacement data set thus generated is suitable for verifying the device status. In particular, since the fluorescence level of the non-fluorescent particle group is susceptible to fluctuations due to, for example, sterilization, it is particularly preferable that the signal intensity data of the non-fluorescent particle group be replaced with the background data for proper verification processing.

As described above, in the verification processing, the pre-replacement data set is acquired. The pre-replacement data set may be a data set of signal intensity of light (specifically, fluorescence) generated by causing the verification sample to flow through the flow channel and irradiating each particle contained in the verification sample with light. Each particle contained in the verification sample may be contained in the sample flow in the laminar flow described above. Then, as described above, the sample flow may be surrounded by the flow of a sheath liquid.

Furthermore, in the verification processing, the background data is acquired. The background data may be a data set of signal intensity of light generated by irradiating the flow channel through which the verification sample is not flowing with light. The background data may be acquired as a result of periodic light detection processing in a state where only the sheath liquid is flowing through the flow channel without the sample (that is, without the sample liquid).

As described above, the pre-replacement data set and the background data may be acquired.

(3-2) Example of verification processing

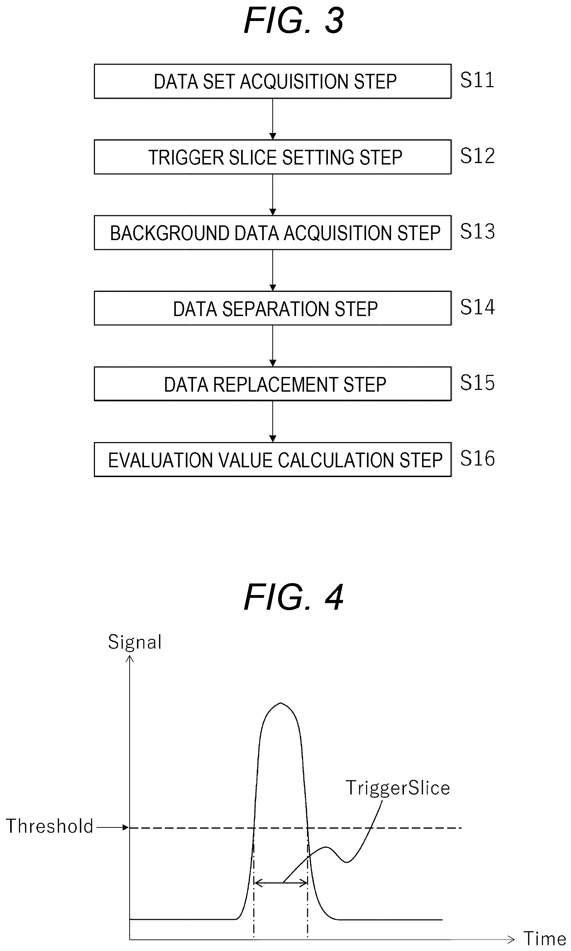

An example of the verification processing will be described below with reference to Fig. 3. The drawing is an example of a flowchart of the verification processing.

As illustrated in the drawing, the verification processing may include a data set acquisition step S11, a trigger slice acquisition step S12, a background data acquisition step S13, a data separation step S14, a data replacement step S15, and an evaluation value calculation step S16. Each step will be described below.

(3-2-1) Data set acquisition step S11

In the data set acquisition step S11, the biological sample analyzer 6100 performs processing of causing the verification sample to flow through the flow channel and acquiring signal intensity data on light generated by irradiating each particle flowing through the flow channel with light. That is, in the data set acquisition step S11, flow cytometry is performed on the verification sample.

For example, the light irradiation unit performs the light irradiation, and the detection unit detects light generated as a result of the light irradiation. The signal intensity data on the detected light is transmitted from the detection unit to the information processing unit.

For example, in a case where the verification sample is a bead population with a plurality of peaks, signal intensity data on fluorescence generated by irradiating each bead contained in the bead population with light is acquired. Examples of the bead population are as described above.

The predetermined fluorescent beads are preferably uniform in size and fluorescence intensity, for example. The predetermined fluorescent beads may be beads that can show fluorescence in a wavelength range of, for example, 400 nm to 800 nm.

The verification sample may be, for example, a sterilized verification sample. The sterilization may cause changes in the fluorescence intensity of the particles contained in the verification sample. It is possible to obtain, by performing the verification processing according to the present disclosure, an accurate evaluation value even in a case where the verification sample that has undergone the changes in the fluorescence intensity is used.

The number of events of the signal acquired in the data set acquisition step may be, for example, greater than or equal to 10,000 events, greater than or equal to 20,000 events, greater than or equal to 30,000 events, greater than or equal to 40,000 events, or greater than or equal to 50,000 events. The above-described number of events enables a more accurate evaluation of the device status.

Furthermore, the number of events may be, for example, less than or equal to 1,000,000 events, less than or equal to 500,000 events, less than or equal to 300,00 events, or less than or equal to 100,00 events from the viewpoint of improving verification processing efficiency.

The number of events may be the number of all events obtained as a result of the light detection processing, or may be the number of pieces of singlet data in all the events. In particular, the number of events may be the number of pieces of singlet data. That is, the data set may be a data set of singlet data.

In the data set acquisition step, processing of acquiring the singlet data may be further performed. Techniques known in the technical field may be used to obtain the singlet data, and for example, data processing by software such as AutoGate may be performed.

In the data set acquisition step, the verification sample may be flowed into the flow channel as the sample liquid. The sample liquid of the verification sample may be surrounded by the sheath liquid, and particularly, the laminar flow in which the sample liquid is surrounded by the sheath liquid is flowed into the flow channel. The biological sample analyzer acquires signal intensity data by irradiating each particle contained in the verification sample flowing as described above with light.

(3-2-2) Trigger slice setting step S12

In the trigger slice setting step S12, the biological sample analyzer sets a trigger slice to be used for the background data to be described later using the data set acquired in the data set acquisition step. The trigger slice setting may be performed by the information processing unit, for example.

For example, as described above, the biological sample analyzer acquires the singlet data from all the events acquired in the data set acquisition step. The biological sample analyzer calculates a median value of the trigger slice (TriggerSlice) from the singlet data. The median value may be set as the trigger slice used for the background data to be described later.

The trigger slice will be described with reference to Fig. 4. The drawing schematically illustrates a change in signal intensity of each event on a graph with the signal intensity of fluorescence detected by the photodetector on the vertical axis and the time on the horizontal axis.

In particle analysis, such as flow cytometry, the photodetector can also detect signals that are not necessary for the analysis. In order to exclude such signals, a threshold for determining whether or not the signal is derived from a target particle is set. In a case where the signal intensity of light generated from a certain particle is greater than or equal to the predetermined threshold, the particle is determined to be the target particle, and in a case where the signal intensity of light generated from a certain particle is less than the predetermined threshold or greater, the particle is determined not to be the target particle. The signal intensity data on the particle determined to be the target particle is used in the analysis, and the signal intensity data on the particle determined not to be the target particle is not used in the analysis. The threshold may be preset in the photodetector. Then, an event that makes a signal greater than or equal to the threshold is generated (that is, the signal intensity data on the target particle) is acquired as data to be analyzed.

As illustrated in the drawing, the trigger slice is a time during which the signal is greater than or equal to the threshold, that is, a time over which the signal crosses the threshold.

In the trigger slice setting step, the trigger slice is acquired from each event included in the singlet data. Then, the median value of the trigger slice is calculated from the acquired trigger slice of each event. The median value thus calculated is used in the background data acquisition step to be described later.

As described above, it is possible to perform, by setting the time called the trigger slice and acquiring the background data in the set trigger slice, the verification processing more properly.

(3-2-3) Background data acquisition step S13

In the background data acquisition step S13, the biological sample analyzer acquires signal intensity data on light generated by irradiating the flow channel through which the verification sample is not flowing with light (that is, background data).

In order to acquire the background data, for example, light irradiation and detection of light generated by the light irradiation are performed in a state where the verification sample is not flowed into the flow channel, but only the sheath liquid is flowed into the flow channel.

For example, the light irradiation unit performs the light irradiation, and the detection unit detects light generated as a result of the light irradiation. The signal intensity data on the detected light is transmitted from the detection unit to the information processing unit.

The light irradiation for acquiring the background data may be performed under the same conditions as the light irradiation performed in the data set acquisition step S11. For example, the light irradiation for acquiring the background data may be the same in laser power as the light irradiation performed in the data set acquisition step S11.

In order to detect the light generated by the light irradiation for acquiring the background data, the trigger slice set in the trigger slice setting step S12 may be used. That is, the signal intensity detected within the time of the trigger slice may be used as one piece of event data.

The detection of the light generated by the light irradiation for acquiring the background data may be performed using an autotrigger. That is, the light detection may be performed at predetermined time intervals.

The number of pieces of signal intensity data that make up the acquired background data may be preferably greater than or equal to [(the number of pieces of event data acquired in the data set acquisition step)/(the number of peaks formed by the verification sample)], preferably greater than [(the number of pieces of event data acquired in the data set acquisition step)/(the number of peaks formed by the verification sample)], more preferably greater than or equal to [(the number of pieces of event data acquired in the data set acquisition step)/(the number of peaks formed by the verification sample) $\times$ 1.1], still more preferably greater than or equal to [(the number of pieces of event data acquired in the data set acquisition step)/(the number of peaks formed by the verification sample) $\times$ 1.2], and still more preferably greater than or equal to [(the number of pieces of event data acquired in the data set acquisition step)/(the number of peaks formed by the verification sample) $\times$ 1.3].

The number of pieces of signal intensity data that make up the acquired background data may be, for example, less than or equal to [(the number of pieces of event data acquired in the data set acquisition step)/(the number of peaks formed by the verification sample) $\times$ 2], less than or equal to [(the number of pieces of event data acquired in the data set acquisition step)/(the number of peaks formed by the verification sample) $\times$ 1.8], or less than or equal to [(the number of pieces of event data acquired in the data set acquisition step)/(the number of peaks formed by the verification sample) $\times$ 1.5].

The number of pieces of signal intensity data that make up the acquired background data may particularly correspond to the number of times of detection using the autotrigger.

For example, the number of particles that form each peak contained in the verification sample such as eight-peak beads is almost the same. Therefore, when the number of pieces of signal intensity data that make up the background data is within the above-described range, the background data becomes data corresponding to data to be replaced in the data replacement step to be described later, which contributes to performing the verification processing properly.

As described above, the number of pieces of signal intensity data acquired in the background acquisition step may be set on the basis of the number of pieces of event data acquired in the data set acquisition step and the number of peaks formed by the verification sample.

For example, it is assumed that the number of events of data acquired using eight-peak beads in the data set acquisition step S11 is 80,000. In this case, the eight-peak beads contains almost the same number of beads for each peak, so the number of events per peak is 80,000/8= 10,000. Considering that there is a slight difference in the number of beads for each peak, 13,333 pieces of signal intensity data increased by 1/3 of 10,000 may be acquired as the background data, for example.

Note that, in a case where the number of pieces of background data is insufficient, the same data may be looped and applied.

Furthermore, in a case where the number of pieces of signal intensity data to be replaced is specified in the data set acquisition step S11 (or before the background data acquisition step S13 is performed), the number of pieces of signal intensity data on the background equal to (or greater than) the number of pieces of signal intensity data to be replaced may be obtained in the background data acquisition step.

In this case, the data separation step for each peak (step similar to the data separation step S14 to be described later) may be performed in the data set acquisition step S11, and the number of pieces of signal intensity data to be replaced may be specified in the data separation step.

For example, in the trigger slice setting step S12, in addition to the acquisition of the singlet data, classification into each particle group described in the data separation step S14 to be described later may be performed. Then, for example, the number of events classified into the particle group with the lowest fluorescence intensity (for example, non-fluorescent beads) may be obtained. The background data may be acquired in the same quantity as the number of events.

As described above, the number of pieces of signal intensity data acquired in the background acquisition step may be set on the basis of the number of pieces of signal intensity data to be replaced with the background data in the data replacement step to be described later.

Furthermore, the signal intensity data to be replaced may be acquired in the data set acquisition step in the same quantity as the signal intensity data acquired in the background data acquisition step.

For example, the number of pieces of signal intensity data acquired in the background data acquisition step may be preset. The data set acquisition step and/or the data separation step may be performed so that the number of events classified into the particle group to be replaced is equal to the set number.

For example, in a case where the number of pieces of signal intensity data acquired in the background data acquisition step is set to 13,333 described above, the data set acquisition step and/or the data separation step may be performed so that the number of events classified into the particle group to be replaced with the background data is up to 13,333.

In the background data acquisition step, the biological sample analyzer causes only the sheath liquid to flow through the flow channel without allowing the sample liquid (verification sample) to flow through the flow channel. That is, in a state where the verification sample is not flowing through the flow channel, irradiating the flow channel with light and detecting light generated by the light irradiation are performed.

In order to cause only the sheath liquid to flow through the flow channel as described above, for example, the biological sample analyzer may stop a pump for supplying the verification sample to the flow channel before performing the background data acquisition step (and after the data acquisition step), or may close a valve provided in the flow channel through which the verification sample is supplied to the flow channel. A state where only the sheath liquid is flowing through the flow channel may be achieved by stopping the pump or the valve.

(3-2-4) Data separation step S14

In the data separation step S14, the biological sample analyzer performs data separation processing on the data set of signal intensity data acquired in the data set acquisition step S11. In the data separation step S14, the data separation processing may be performed on the singlet data in the data set of signal intensity data acquired in the data set acquisition step S11. The data separation step may be performed by the information processing unit, for example.

In the data separation step S14, the signal intensity data on the verification sample is separated into data of each of the plurality of types of particle groups having varying fluorescence intensity levels. For example, in a case where the verification sample has eight peaks, the data set of high signal intensity data acquired for the verification sample is separated into data of eight particle groups corresponding to each peak. The separation is useful for identifying the signal intensity data to be replaced with the background data.

As described above, the biological sample analyzer may perform the data separation step to identify the signal intensity data to be replaced with the background data in the data replacement step to be described later. It is possible to obtain, by replacing the signal intensity data thus identified with the background data, a signal intensity data set useful for obtaining an accurate evaluation value.

In the data separation step, the signal intensity data acquired in the fluorescent dye channel can be clustered for each event. The clustering allows each event to be classified into any one of the plurality of types of particle groups. For example, in a case where the verification sample has eight peaks, each piece of event data included in the data set of high signal intensity data acquired for the verification sample is clustered into one of eight groups. As described above, the biological sample analyzer may be configured to perform data separation through the clustering processing in the data separation step.

As the fluorescent dye channel used for the clustering, for example, one or more fluorescent dye channels capable of satisfactorily separating the plurality of types of particle groups may be selected.

When sterilization is performed on the verification sample, level fluctuations in the acquired fluorescence signal intensity may occur in all the fluorescent dye channels. In some fluorescent dye channels, two peaks in the region of low signal intensity may merge, making it difficult to separate data between these two peaks. Therefore, in the data separation step, clustering is performed for each event using fluorescent dye channels with good separation performance to classify the events into the plurality of types of particle groups. For example, eight-peak beads are classified into eight particle groups Dim1 to Dim8 through the clustering in the data separation step.

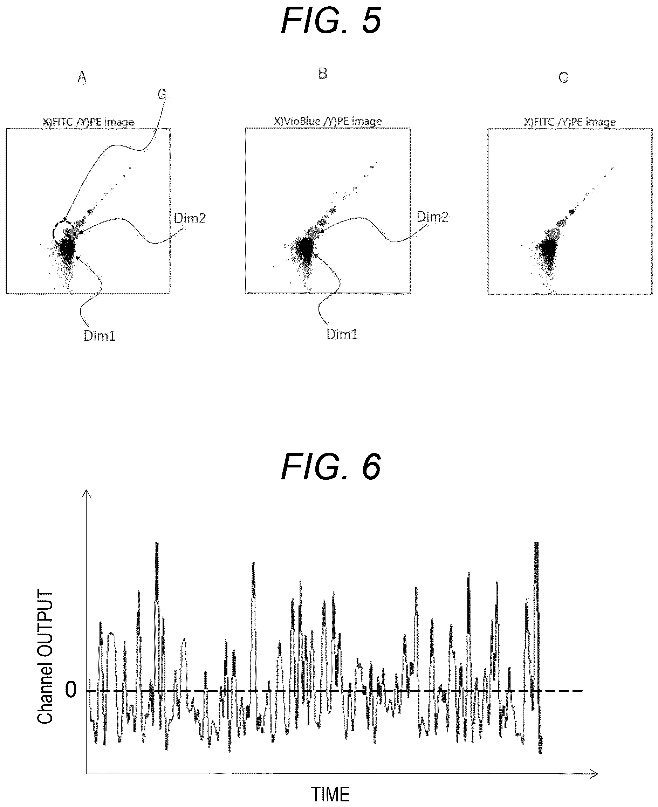

The data separation will be described with reference to Fig. 5. A to C of the drawing are two-dimensional plot data where each event is plotted on the basis of the signal intensity of eight-peak beads in two fluorescent dye channels. In A of the drawing, the X axis represents the signal intensity in the FITC channel, and the Y axis represents the signal intensity in the PE channel. In B of the drawing, the X axis represents the signal intensity in the VioBlue channel, and the Y axis represents the signal intensity in the PE channel. In C of the drawing, the data separation itself is performed using the VioBlue/PE channels with good separation performance, and the plot data is displayed on the basis of FITC/PE.

In A of the drawing, some events to be classified into Dim1 are classified into Dim2, and some events to be classified into Dim2 are classified into Dim1. That is, the separation between Dim1 and Dim2 is not properly performed. On the other hand, as for B of the drawing, the separation is properly performed and C corresponds to a two-dimensional plot created using FITC/PE on the basis of the clustering information in the separation of B, and as for C as well, the separation is properly performed as in B, which is better than A.

On the basis of such plot data, the VioBlue channel and the PE channel may be selected as the fluorescent dye channels to be used in the data separation step.

It is possible to obtain an accurate evaluation value by performing clustering using the fluorescent dye channel that enables proper separation as described above.

In one embodiment, the fluorescent dye channel may be selected on the basis of, for example, two-dimensional plot data with the signal intensity in one fluorescent dye channel on the X-axis and the signal intensity of another fluorescent dye channel on the Y-axis.

In order to perform the selection, the biological sample analyzer may generate the two-dimensional plot data described above using, for example, the signal intensity data acquired using any two fluorescent dye channels among a plurality of fluorescent dye channels included in the detection unit of the biological sample analyzer. For example, the two-dimensional plot data may be created for each pair of the plurality of fluorescent dye channels.

It is therefore possible for the user of the biological sample analyzer to select the fluorescent dye channel to be used in the data separation step.

Furthermore, on the basis of the generated two-dimensional plot data, the biological sample analyzer may select the fluorescent dye channel to be used in the data separation step.

In another embodiment, the fluorescent dye channel may be selected on the basis of a separation index (SI) of each fluorescent dye channel. For example, the biological sample analyzer may calculate the SI of each fluorescent dye channel, and select two fluorescent dye channels with the highest SIs from among the calculated SIs. The clustering may be performed using the signal intensity data acquired from the selected two fluorescent dye channels.

For example, the SI may be calculated using the signal intensity of two types of particle groups that produce two adjacent peaks among the signal intensity data acquired from any two fluorescent dye channels. For example, the SI can be calculated using the median and standard deviation of the signal intensity of each of the two types of particle groups.

For example, it is assumed that, the two types of particle groups among the plurality of types of particle groups include a first particle group Dim1 and a second particle group Dim2, and the second particle group Dim2 is higher in signal intensity than the first particle group Dim1. In this case, the SI is expressed by the following formula:

$$SI = (Dim2\_Median - Dim1\_Median)/(rSD\_Dim2 + rSD\_Dim1).$$

In this formula, Dim2_Median represents the median of the signal intensity of the second particle group Dim2, and Dim1_Median represents the median of the signal intensity of the second particle group Dim1. Furthermore, rSD_Dim2 represents the standard deviation of the signal intensity of the second particle group Dim2, and rSD_Dim1 represents the standard deviation of the signal intensity of the first particle group Dim1.

The clustering processing may be performed using a known method such as the k-means method. An example of a more specific method of the clustering processing is disclosed in, for example, WO 2016/185755A.

For example, in the clustering processing, a first center intensity of the signal intensity data of each particle group may be calculated on the basis of the signal intensity data acquired as a result of light detection, and the clustering may be repeatedly performed on the basis of the first center intensity. This allows each event to be properly classified into the corresponding particle group.

(3-2-5) Data replacement step S15

In the data replacement step S15, the biological sample analyzer replaces the signal intensity data on light generated by irradiating any one type of particle group in the data set acquired in the data set acquisition step S11 with the signal intensity data acquired by irradiating the flow channel through which no particles are flowing with light. That is, as described above, the signal intensity data on any one type of particle group in the pre-replacement data set is replaced with the background data. As a result, the post-replacement data set including the background data is generated. The data replacement step may be performed by the information processing unit, for example.

The signal intensity data to be replaced in the data replacement step may be any one of the pieces of signal intensity data of the plurality of types of particle groups separated in the data separation step. In particular, the signal intensity data to be replaced in the data replacement step may be the signal intensity data of a particle group with the lowest signal intensity. That is, the biological sample analyzer may be configured to replace the signal intensity data of a particle group with the lowest fluorescence level among the plurality of types of particle groups with the signal intensity data acquired by irradiating the flow channel through which no particles are flowing with light.

Since a particle group with a lower fluorescence level is particularly susceptible to fluctuations in fluorescence level, the effect of the data replacement according to the present disclosure becomes particularly pronounced by replacing the signal intensity data of the particle group.

In particular, it is preferable that the pre-replacement data set include the signal intensity data of the non-fluorescent particle group as described above, and in the data replacement step, the signal intensity data of the non-fluorescent particle group be replaced with the background data. That is, in a case where the sample containing the plurality of types of particle groups contains one type of non-fluorescent particle group and one or more types of fluorescent particle groups, the biological sample analyzer may be configured to replace the signal intensity data of the one type of non-fluorescent particle group with the signal intensity data acquired by irradiating the flow channel through which no particles are flowing with light in the data replacement step.

Since the non-fluorescent particle group is particularly susceptible to fluctuations in fluorescence level, the effect of the data replacement according to the present disclosure becomes particularly pronounced by replacing the signal intensity data of the particle group.

(3-2-6) Evaluation value calculation step S16

In the evaluation value calculation step S16, the biological sample analyzer calculates one or more evaluation values for evaluating the status of the device using the post-replacement data set obtained in the data replacement step. The one or more evaluation values may include, for example, at least any one of the precision evaluation value of the device, the linearity evaluation value of the device, of the sensitivity evaluation value of the device. The evaluation value calculation step may be performed by the information processing unit, for example.

It is preferable that the precision evaluation value be calculated in the evaluation value calculation step. The precision evaluation value may be, for example, MESF. It is preferable that the precision evaluation value (specifically MESF) be calculated using the background data in the post-replacement data set, specifically using only positive signal intensity data in the background data.

The output of each fluorescent dye channel is clamped to ensure that the long-term average of the signal intensity data on the background when no sample is flowing becomes zero. The background increases when the optical noise or electrical noise becomes larger. Furthermore, the background shows swings like alternating current (AC), as shown in Fig. 6. When the level of the AC-like swings increases, statistics (such as a mean value) based only on positive values increase, and can be used as an evaluation value for the status of the device.

In the evaluation value calculation step, in addition to the precision evaluation value, the linearity evaluation value and/or the sensitivity evaluation value of the device may be calculated. The linearity evaluation value may be, for example, Linearity. The sensitivity evaluation value may be, for example, Q value and/or B value. The Q value represents detection efficiency. The B value represents background.

The details and calculation methods of these evaluation values will be described below on the basis of a case where eight-peak beads are used. For other verification samples, a person skilled in the art can properly calculate each evaluation value.

(a) Definitions

Particle group Dim1: a particle group whose fluorescence intensity belongs to the lowest peak
Particle group Dim2: a particle group whose fluorescence intensity belongs to the second lowest peak
Particle group Dim3: a particle group whose fluorescence intensity belongs to the third lowest peak
Particle group Dim4: a particle group whose fluorescence intensity belongs to the fourth lowest peak
Particle group Dim5: a particle group whose fluorescence intensity belongs to the fifth lowest peak
Particle group Dim6: a particle group whose fluorescence intensity belongs to the sixth lowest peak
Particle group Dim7: a particle group whose fluorescence intensity belongs to the seventh lowest peak
Particle group Dim8: a particle group whose fluorescence intensity belongs to the eighth lowest peak
(Dim1 to Dim8 are also referred to as Peak1 to Peak8, respectively.)
MFI: mean fluorescence intensity (a value obtained by converting the maximum and minimum mean fluorescence signal intensities into 1 and 0, respectively)
MEF2 to MEF8: reference MESF values of Dim2 to Dim8
MESF: Molecules of Equivalent Soluble Fluorochromes (the number of fluorescence molecules per particle)
Mean values of Dim1 to Dim8: MFI1 to MFI8
Log values of MFI1 to MFI 8: LogMFI1 to LogMFI8
Reference values of Dim1 to Dim8: MEF1 to MEF8
Log values of MEF2 to MEF8: LogMEF2 to LogMEF8
MESF value calculated for Dim1: MESF
MESF of Dim2 to Dim4: MESF2 to MESF4
Coefficients of variation of Dim2 to Dim8: CV2 to CV8
Standard deviations of Dim2 to Dim8: SD2 to SD8 [0077]

(b) Linearity

$R^2$ (coefficient of determination) is determined using LogMFI2 to LogMFI8 and LogMEF2 to LogMEF8, and R obtained by taking the square root of $R^2$ is defined as linearity.
The closer the linearity is to 100%, the better.
(c) Precision (Molecules of Equivalent Soluble Fluorochromes (MESF), also referred to as fluorescence detection sensitivity)

[1] Regression line is determined using LogMFI2 to LogMFI8 and LogMEF2 to LogMEF8.
[2] Fluorescence detection sensitivity (MESF) is determined using MFI1 from the regression line.

The closer the fluorescence detection sensitivity (MESF) is to the reference value (usually 0) of Dim1, the better.

(d) Sensitivity (Q value and B value)

MESF2 to MESF4 of Dim2 to Dim4 are determined.
$(SD2)^2 = ((CV2)^2 - (CV8)^2) \times (MESF2)^2$ is determined, and $(SD3)^2$ and $(SD4)^2$ are also determined in a similar manner.
MESF2 to MESF4 are plotted on the horizontal axis and $(SD2)^2$ to $(SD4)^2$ are plotted on the vertical axis to determine the regression line, and the slope and intercept of the regression line are denoted as a and b, respectively.
The Q value is the reciprocal of the slope of the regression line (1/a).
The B value is the ratio of the slope to the intercept of the regression line (b/a).
In order to calculate the one or more evaluation values, the molecules of equivalent fluorochrome (MEF) of the verification sample may be used. The MEF may be held by the biological sample analyzer in advance, and may be stored in the information processing unit (specifically, the storage unit) of the biological sample analyzer, for example.
In one embodiment, the verification sample used in the present disclosure may be a sample with varied fluorescence levels for each peak as described above, and may be, for example, a sterilized verification sample. In this case, the MEF used for calculating the one or more evaluation values is the MEF of the verification sample with varied fluorescence levels, specifically the MEF of the sterilized verification sample.
In this embodiment, as the MEF used for calculating the one or more evaluation values, the MEF of the verification sample with unvaried fluorescence levels is not suitable for use, that is, the MEF of the unsterilized verification sample is not suitable for use.

Therefore, the biological particle analyzer of the present disclosure may hold the MEF of the verification sample in advance, or may acquire the MEF of the verification sample as follows.

First, the signal intensity data set of the reference sample is acquired as described in the data set acquisition step S11. The signal intensity data set of the reference sample is separated for each type of particle group as described in the data separation step S14, and the MFI of each of the plurality of types of particle groups (specifically, each fluorescent particle group) is acquired.

Similarly, for the verification sample as well, the signal intensity data set is acquired, and then the MFI of each of the plurality of types of particle groups (specifically, each fluorescent particle group) is acquired.

For each particle group, the MFI of each particle group of the verification sample is calculated from the ratio between the MFI of the reference sample and the MFI of the verification sample. Specifically, the MFI is calculated using the following formula:

(MEF of a certain particle group of the verification sample) = (MEF of a certain particle group of the reference sample) × (MFI of the certain particle group of the verification sample)/(MFI of the certain particle group of the reference sample).

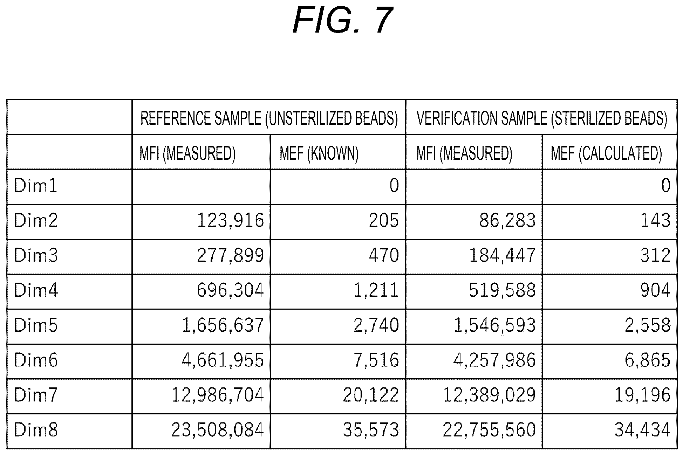

Fig. 7 shows examples of (measured) MFI and (known) MEF of the reference sample and examples of (measured) MFI and (calculated) MEF of the verification sample. For example, as for Dim2, in order to calculate the MEF of the verification sample, the MEF of 205 × 86283/123916 = 143 is calculated using the MFI and MEF of Dim2 of the reference sample and the MFI of the verification sample.

It is preferable that the MEF of the verification sample be applied to the verification sample of the same lot. The biological sample analyzer may acquire the MEF of the verification sample from, for example, a two-dimensional code (such as a QR code (registered trademark)) or the like via a network.

(3-3) Other steps

The biological sample analyzer may perform an output step of outputting the calculated evaluation value to a display device after the evaluation value calculation step. It is possible to prompt, by displaying the evaluation value in the output step, the user of the device to determine whether or not to perform the biological sample analysis and to determine whether or not to calibrate the biological sample analyzer. The biological sample analyzer may cause the display device to display a screen for prompting such determinations.

The biological sample analyzer may determine whether or not to perform the biological sample analysis on the basis of the calculated evaluation value after the evaluation value calculation step. That is, whether or not the device performs the biological sample analysis after the verification processing may be automatically determined. For the determination, the biological sample analyzer may have a predetermined threshold in advance for determining whether or not to allow the biological sample analysis to be performed, and the biological sample analysis may be performed on the basis of whether or not the value is greater than or equal to or less than or equal to the threshold.

(4) Examples

Verification processing of verifying the device status of the cell sorter was performed using commercially available eight-peak beads without sterilization. Similarly, the eight-peak beads were sterilized, and the verification processing of verifying the device status of the cell sorter was performed in a similar manner. The eight-peak beads contain one type of non-fluorescent beads and seven types of fluorescent beads, and form eight peaks as a result of flow cytometry. As described in the above (2), the cell sorter includes the light irradiation unit, the detection unit, the information processing unit, and the sorting unit.

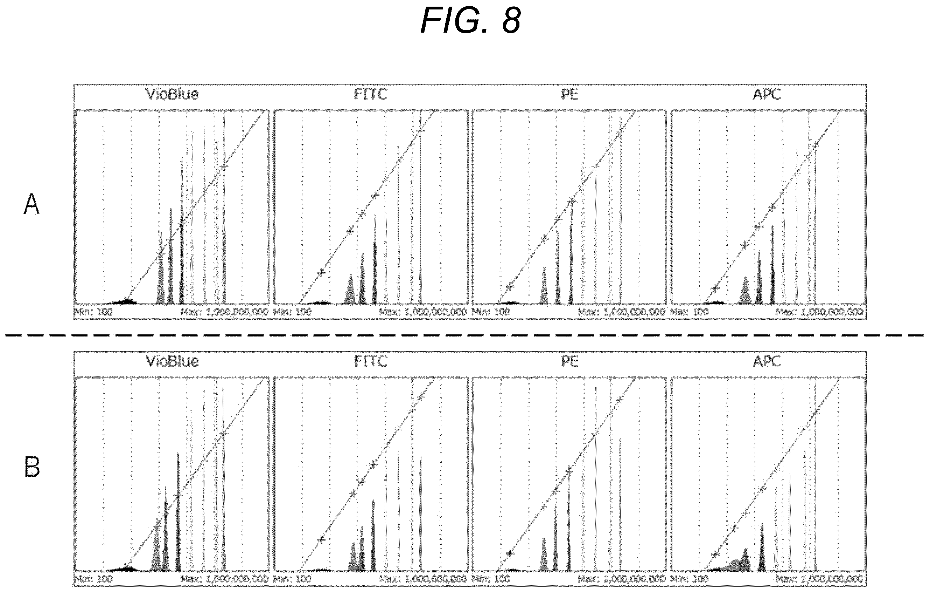

First, the results of fluorescence separation of unsterilized eight-peak beads and the results of fluorescence separation of sterilized eight-peak beads are shown in Fig. 8. The results of fluorescence separation of unsterilized eight-peak beads are shown in A on the upper side of the drawing, and the results of fluorescence separation of sterilized eight-peak beads are shown in B on the lower side of the drawing. As shown in these results, the unsterilized eight-peak beads were well-separated in terms of fluorescence. On the other hand, as for the sterilized eight-peak beads, for example, the peak of the non-fluorescent bead and the adjacent peak are close to each other, preventing these peaks from being separated.

Furthermore, for the unsterilized eight-peak beads and the sterilized eight-peak beads, their respective evaluation values for verifying the device status were calculated using the obtained signal intensity data set. The calculated evaluation values include the linearity evaluation value (Linearity), the precision evaluation value (MESF), and the sensitivity evaluation value (Q value and B value).

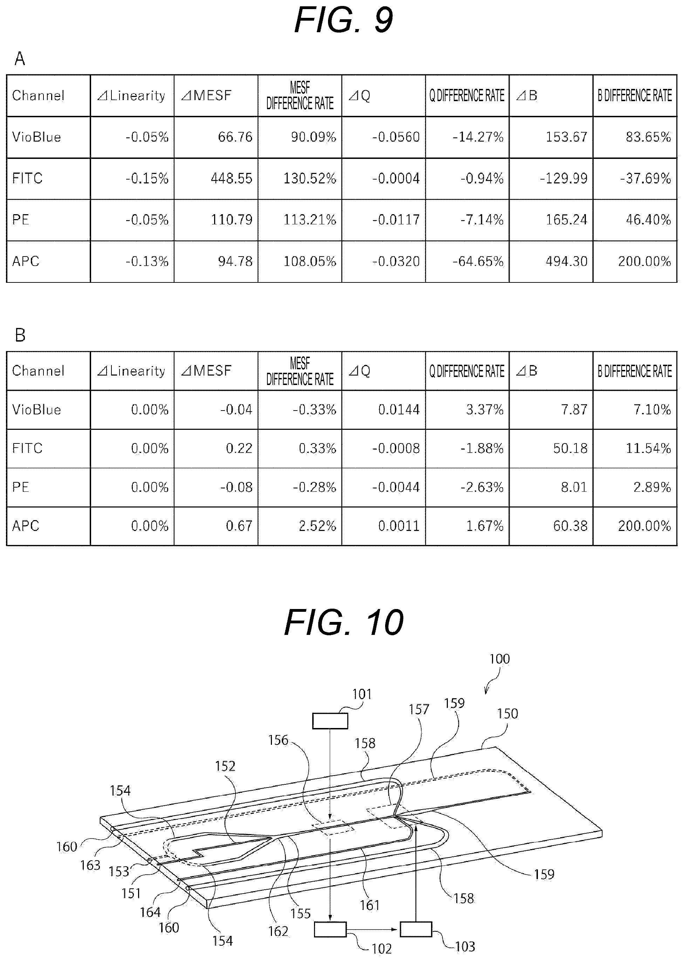

Differences (∆X: X represents each evaluation value) and difference rate (X difference rate: X represents each evaluation value) between the evaluation values obtained using the unsterilized eight-peak beads and the evaluation values obtained using the sterilized eight-peak beads are shown in the upper table A of Fig. 9. The differences and the difference rates were calculated using the following formulas:

∆X = (evaluation value of sterilized eight-peak beads) - (evaluation value of unsterilized eight-peak beads) ;

and X difference rate (%) = ((evaluation value of sterilized eight-peak beads) - (evaluation value of unsterilized eight-peak beads))/((evaluation value obtained using sterilized eight-peak beads) + (evaluation value obtained using unsterilized eight-peak beads)/2) $\times$ 100.

As shown in the drawing, the linearity evaluation value of the sterilized eight-peak beads differs from that of the unsterilized eight-peak beads and decreases. The precision evaluation value of the sterilized eight-peak beads differs from that of the unsterilized eight-peak beads and increases. The sensitivity evaluation value of the sterilized eight-peak beads differs from that of the unsterilized eight-peak beads and decreases or increases.

As described above, the eight-peak beads undergo fluctuations in fluorescence level due to sterilization, and the evaluation value for evaluating the device status changes accordingly.

Next, the validation processing according to the present disclosure was performed using signal intensity data sets obtained for both the unsterilized eight-peak beads and the sterilized eight-peak beads. That is, the evaluation values were calculated using the post-replacement data sets obtained by replacing the signal intensity data of the non-fluorescent beads in the signal intensity data set with the background data of the cell sorter. The calculated evaluation values include the linearity evaluation value (Linearity), the precision evaluation value (MESF), and the sensitivity evaluation value (Q value and B value) in a similar manner as described above.

Differences and difference rates between the evaluation values obtained using the unsterilized eight-peak beads and the evaluation values obtained using the sterilized eight-peak beads are shown in the lower table B of Fig. **9.** As shown in the drawing, the linearity evaluation value of the sterilized eight-peak beads is the same as that of the unsterilized eight-peak beads, and is significantly improved as compared with that shown in Table A of the drawing. As shown in the drawing, the precision evaluation value of the sterilized eight-peak beads is almost the same as that of the unsterilized eight-peak beads, and is significantly improved as compared with that shown in Table A of the drawing. As shown in the drawing, the sensitivity evaluation value of the sterilized eight-peak beads is almost the same as that of the unsterilized eight-peak beads, and is significantly improved as compared with that shown in Table A of the drawing.

The above shows that, by performing the verification processing according to the present disclosure, the device status is properly evaluated even in a case where the sterilized verification sample is used.

Furthermore, it is considered that the use of the MEF calculated as described above in addition to the application of the background data contributes to the improvement of linearity and sensitivity.

Furthermore, it is considered that the use of the MEF calculated as described above and the use of fluorescent dye channels suitable for separation in addition to the application of the background data contributes to the improvement of precision.

(5) Configuration example of biological particle sorting device

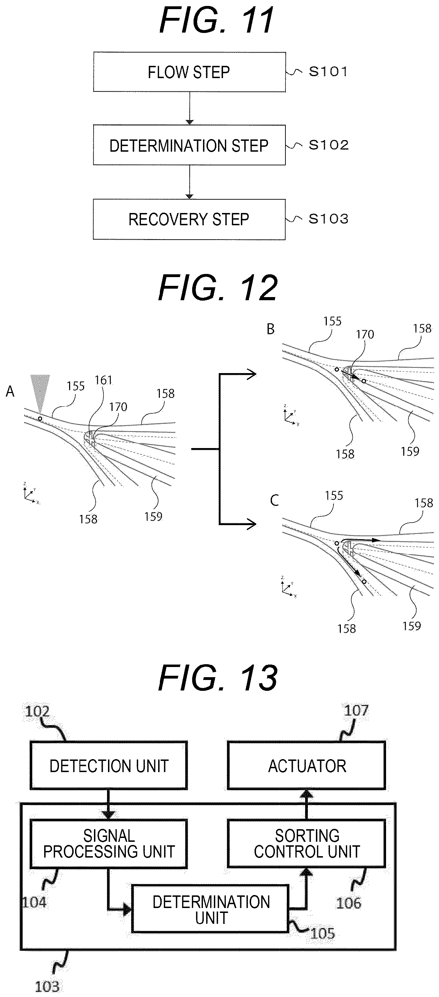

In one embodiment, the biological sample analyzer of the present disclosure may be configured as a biological particle sorting device, such as a cell sorting device. The biological particle sorting device may be a device that analyzes and/or sorts out biological particles in a microchip without forming droplets. The biological particle sorting device may be configured to perform the verification processing described above. This embodiment will be described below with reference to Figs. 10 and 11.

Fig. 10 illustrates a schematic diagram of a configuration example of a biological particle sorting microchip, and a configuration example of a biological particle analyzer including the microchip. Fig. 11 illustrates an example of a flowchart of a biological particle sorting operation performed by the biological particle analyzer.

The biological particle sorting microchip 150 illustrated in Fig. 10 includes a sample liquid flow channel 152 and a sheath liquid flow channel 154 that joins the sample liquid flow channel 152 at a junction 162. The biological particle sorting microchip 150 further includes a sample liquid inlet 151 and a sheath liquid inlet 153.

Note that, in Fig. 10, a part of the sheath liquid flow channel 154 is indicated by a dotted line. The part indicated by the dotted line is located in a position lower than that of the sample liquid flow channel 152 indicated by a solid line (position displaced in an optical axis direction to be described later), and the flow channel indicated by the dotted line and the flow channel indicated by the solid line are not communicated with each other in a position where the flow channels intersect. Furthermore, in Fig. 10, the sample liquid flow channel 152 is illustrated to bend twice between the sample liquid inlet 151 and the junction 162, which facilitates distinction between the sample liquid flow channel 152 and the sheath liquid flow channel 154. The sample liquid flow channel 152 may be formed linearly without bending in this manner between the sample liquid inlet 151 and the junction 162.

In the biological particle sorting operation, a sample liquid containing biological particles is introduced from the sample liquid inlet 151 into the sample liquid flow channel 152, and a sheath liquid not containing the biological particles is introduced from the sheath liquid inlet 153 into the sheath liquid flow channel 154.

The biological particle sorting microchip 150 includes a joined flow channel 155 including the junction 162 at one end.

The sample liquid and the sheath liquid join at the junction 162, and then flow through the joined flow channel 155 toward a particle sorting unit 157. In particular, the sample liquid and the sheath liquid join at the junction 162 to form, for example, a laminar flow in which the sample liquid is surrounded by the sheath liquid. Preferably, in the laminar flow, the biological particles are arrayed substantially in a line. The flow channel structure in which the sample liquid flow channel 152 and two sheath liquid flow channels 154 join at the junction 162 and that includes the joined flow channel 155 having one end serving as the junction 162, forms the laminar flow including the biological particles that flow substantially in a line. Therefore, when a detection area 156 to be described below is irradiated with light, light generated by irradiating one biological particle with light and light generated by irradiating another biological particle with light can be easily distinguished from each other.

The biological particle sorting microchip 150 further includes the particle sorting unit 157 at the other end of the joined flow channel 155. Fig. 12 is an enlarged view of the particle sorting unit 157. As illustrated in A of Fig. 12, at the other end, the joined flow channel 155 is connected to a biological particle recovery flow channel 159 via a connection flow channel 170. As illustrated in A of Fig. 12, the joined flow channel 155, the connection flow channel 170, and the biological particle recovery flow channel 159 may be coaxial with each other.

In a case where a recovery target particle flows to the particle sorting unit 157, as illustrated in B of Fig. 12, a flow from the joined flow channel 155 through the connection flow channel 170 to enter the biological particle recovery flow channel 159 is formed, and the recovery target particle is recovered into the biological particle recovery flow channel 159. In this manner, the recovery target particle flows through the connection flow channel 170 to the biological particle recovery flow channel 159.

In a case where the biological particle that is not the recovery target particle flows to the particle sorting unit 157, the biological particle that is not the recovery target particle flows to a branching flow channel 158 as illustrated in C of Fig. 12. In this case, the flow to enter the biological particle recovery flow channel 159 is not formed.

As illustrated in Fig. 10, the biological particle recovery flow channel 159 is formed so as to extend linearly from the particle sorting unit 157, make a U-turn, then reach the same surface as a surface on which the sample liquid inlet 151 and the sheath liquid inlet 153 are formed. The liquid that flows through the biological particle recovery flow channel 159 is discharged out of the chip from a recovery flow channel terminal 163.

As illustrated in Fig. 10, the two branching flow channels 158 are also formed so as to extend linearly from the particle sorting unit 157, make a U-turn, and then reach the same surface as the surface on which the sample liquid inlet 151 and the sheath liquid inlet 153 are formed. The liquid that flows through the branching flow channel 158 is discharged out of the chip from a branching flow channel terminal 166.

In Fig. 10, a display method of the biological particle recovery flow channel 159 is changed from a solid line to a dotted line at the U-turn. This change indicates that the position in the optical axis direction changes on the way. By changing the position in the optical axis direction in this manner, the biological particle recovery flow channel 159 is not communicated with the branching flow channel 158 in a portion intersecting with the branching flow channel 158.

Both the recovery flow channel terminal 163 and the two branching flow channel terminals 166 are formed on the surface on which the sample liquid inlet 151 and the sheath liquid inlet 153 are formed. Moreover, an introduction flow channel inlet 164 for introducing a liquid into an introduction flow channel 161 is also formed on the surface. In this manner, the biological particle sorting microchip 150 has all of the inlets from which the liquid is introduced and the outlets from which the liquid is discharged formed on one surface. This facilitates the attachment of the chip to a biological particle analyzer 100. For example, as compared with a case where the inlets and/or outlets are formed on two or more surfaces, connection between flow channels provided in the biological particle analyzer 100 and the flow channels of the biological particle sorting microchip 150 becomes easy.

As illustrated in Figs. 10 and 12, the biological particle sorting microchip 150 includes the introduction flow channel 161 for introducing the liquid into the connection flow channel 170.

By introducing the liquid from the introduction flow channel 161 into the connection flow channel 170, the connection flow channel 170 is filled with the liquid. It is therefore possible to prevent unintended biological particles from entering the biological particle recovery flow channel 159.

The biological particle sorting microchip 150 includes the two branching flow channels 158 connected to the joined flow channel 155 at the other end of the joined flow channel 155. In this manner, in the biological particle sorting microchip used in the present technology, the joined flow channel may be branched into the connection flow channel and the at least one branching flow channel.

The biological particles other than the recovery target particle flow to either of the two branching flow channels 158 without entering the biological particle recovery flow channel 159.

Furthermore, as illustrated in Fig. 10, the biological particle sorting microchip 150 forms a part of the biological particle analyzer 100 including a light irradiation unit 101, a detection unit 102, and a control unit 103 in addition to the microchip. The light irradiation unit 101, the detection unit 102, and the control unit 103 correspond to the light irradiation unit 6101, the detection unit 6102, and the information processing unit 6103 described in the above (2), respectively, and the descriptions thereof also applies to the present configuration example. As illustrated in Fig. 13, the control unit 103 of the biological particle analyzer 100 may include a signal processing unit 104, a determination unit 105, and a sorting control unit 106.

As illustrated in Fig. 11, the biological particle sorting operation using the biological particle sorting microchip 150 includes a flow step S101 of allowing the liquid containing the biological particles to flow through the joined flow channel 155, a determination step S102 of determining whether or not the biological particles flowing through the joined flow channel 155 are the recovery target particles, and a recovery step S103 of recovering the recovery target particles into the biological particle recovery flow channel 159. Each step will be described below.

(5-1) Flow step

In the flow step S101, the sample liquid containing the biological particles and the sheath liquid not containing the biological particles are introduced from the sample liquid inlet 151 and the sheath liquid inlet 153 into the sample liquid flow channel 152 and the sheath liquid flow channel 154, respectively.

The sample liquid and the sheath liquid join at the junction 162 to form, for example, the laminar flow in which the sample liquid is surrounded by the sheath liquid. Preferably, in the laminar flow, the biological particles are arrayed substantially in a line. That is, in the flow step S101, the laminar flow containing the biological particles that flow substantially in a line may be formed.

In this manner, in the flow step S101, the liquid containing the biological particles is allowed to flow through the joined flow channel 155, specifically as the laminar flow. The liquid flows through the joined flow channel 155 from the junction 162 toward the particle sorting unit 157.

(5-2) Determination step

In the determination step S102, it is determined whether or not the biological particles flowing through the joined flow channel 155 are the recovery target particles. The determination may be performed by the determination unit 105. The determination unit 105 may perform the determination on the basis of the light generated when the light irradiation unit 101 irradiates the biological particles with light.

The signal processing unit 104 included in the control unit 103 may process a waveform of the digital electric signal obtained by the detection unit 102 to generate information (data) regarding the characteristics of the light used for the determination by the determination unit 105. As the information regarding the characteristics of the light, the signal processing unit 104 may acquire, for example, one, two, or all of the width of the waveform, the height of the waveform, and the area of the waveform from the waveform of the digital electrical signal. Furthermore, the information regarding the characteristics of the light may include, for example, the time when the light is detected. The processing of the signal processing unit 104 described above may be performed particularly in the embodiment in which the scattered light and/or fluorescence is detected.

On the basis of the light generated by irradiating the biological particles flowing through the flow channel with light, the determination unit 105 included in the control unit 103 determines whether or not the biological particles are the recovery target particles.

(5-3) Recovery step

In the recovery step S103, the biological particles determined to be the recovery target particles in the determination step S102 are recovered into the biological particle recovery flow channel 159. The recovery step S103 is performed in the particle sorting unit 157 in the microchip 150. In the particle sorting unit 157, the laminar flow flowing through the joined flow channel 155 separately flows to the two branching flow channels 158. The particle sorting unit 157 illustrated in Fig. 10 includes the two branching flow channels 158, but the number of the branching flow channels is not limited to two. The particle sorting unit 157 may be provided with, for example, one or a plurality of (for example, two, three, or four) branching flow channels. The branching flow channel may be formed to branch into a Y shape on one plane as illustrated in Fig. 10, or may be formed to branch three-dimensionally.

In the recovery step S103, due to the pressure fluctuations in the biological particle recovery flow channel 159, the recovery target particles are recovered into the biological particle recovery flow channel through the connection flow channel. The recovery may be performed, for example, by generating the negative pressure in the biological particle recovery flow channel 159 as described above. The negative pressure may be generated, for example, when a wall that defines the biological particle recovery flow channel 159 is deformed by an actuator 107 (specifically, a piezo actuator) attached to an outer side of the microchip 150. The negative pressure may form the flow entering the biological particle recovery flow channel 159. In order to generate the negative pressure, the actuator 107 may be attached to the outside of the microchip 150, for example, so that the wall of the biological particle recovery flow channel 159 can be deformed. The negative pressure may be generated by the deformation of the wall that changes an inner space of the biological particle recovery flow channel 159. The actuator 107 may be, for example, a piezo actuator. When the recovery target particles are sucked into the biological particle recovery flow channel 159, the sample liquid that forms the laminar flow or the sample liquid and the sheath liquid that form the laminar flow may also flow to the biological particle recovery flow channel 159. In this manner, the recovery target particles are sorted in the particle sorting unit 157 and recovered into the biological particle recovery flow channel 159.

The connection flow channel 170 is provided with the introduction flow channel 161 in order to prevent the biological particles that are not the recovery target particles from entering the biological particle recovery flow channel 159 through the connection flow channel 170. The liquid is introduced into the connection flow channel 170 from the introduction flow channel 161. By the introduction of the liquid, the connection flow channel 170 is filled with the liquid. Moreover, since a flow from the connection flow channel 170 to the joined flow channel 155 is formed by a part of the liquid, it is possible to prevent the biological particles other than the recovery target particles from entering the biological particle recovery flow channel 159. The liquid that forms the flow from the connection flow channel 170 to the joined flow channel 155 flows, by the flow of the liquid flowing through the joined flow channel 155 to the branching flow channel 158, through the branching flow channel 158 in a manner similar to the liquid without flowing through the joined flow channel 155.

Note that, the rest of the liquid introduced into the connection flow channel 170 flows to the biological particle recovery flow channel 159. Therefore, the biological particle recovery flow channel 159 may be filled with the liquid.

The flow to the branching flow channel 158 may be discharged out of the microchip at the branching flow channel terminal 160. Furthermore, the recovery target particles recovered into the biological particle recovery flow channel 159 may be discharged out of the microchip at the recovery flow channel terminal 163. A container may be connected to the recovery flow channel terminal 163 via a flow channel such as a tube. The recovery target particles may be recovered into the container.

2. Second embodiment (biological sample analysis system)

The present disclosure further provides a biological sample analysis system configured to perform the verification processing described in the above 1. That is, the system includes an information processing unit configured to perform information processing using signal intensity data on light generated by irradiating a flow channel through which particles are flowing with light, in which the information processing unit may be configured to perform a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

In addition to the information processing unit, the biological sample analysis system may include the light irradiation unit and the detection unit described in the above 1. and may further include a sorting unit. These components may be provided in one device or may be provided in a plurality of devices in a distributed manner. For example, the biological sample analysis system may include an information processing device configured as the information processing unit. The biological sample analysis system may include an analyzer including the light irradiation unit and the detection unit (and the sorting unit) in addition to the information processing device. These devices may be connected to each other in a wired or wireless manner. Furthermore, these devices may be connected to each other via a network.

The system may perform the verification processing according to the present disclosure as follows, for example.

In the data set acquisition step S11 of the flowchart in Fig. 3 described in the above 1., the analyzer (specifically, the light irradiation unit and the detection unit) constituting the biological sample analysis system performs light irradiation and detection of light generated by the light irradiation, and the information processing device acquires a signal intensity data set on the light.

In the trigger slice setting step S12, the information processing device performs the trigger slice setting. The information processing device transmits the set trigger slice to the analyzer.

In the background data acquisition step S13, the analyzer performs light irradiation and light detection for background data acquisition using the trigger slice, and transmits the background data detected through the light detection to the information processing device. The information processing device receives the background data.

The information processing device performs the data separation step S14, the data replacement step S15, and the evaluation value calculation step S16 using the acquired signal intensity data set and background data.

3.Third embodiment (verification method of biological sample analyzer and program for executing verification method)

The present disclosure further provides a verification method for verifying the status of the biological sample analyzer including performing the verification processing described in the above 1. That is, the verification method includes a data replacement step of causing a sample containing a plurality of types of particle groups having varying fluorescence intensity levels to flow into a flow channel, and replacing signal intensity data on light generated by irradiating, with light, on any one type of particle group in a data set of signal intensity data on light generated by irradiating, with light, particles flowing through the flow channel with signal intensity data acquired by irradiating the flow channel through which no particles are flowing with light. The verification method may be performed as described in the above 1. with reference to Fig. 3, for example, and the description also applies to the verification method.

The present disclosure further provides a program for causing the biological sample analyzer or the information processing device to perform the verification method. The program may be stored in, for example, the biological sample analyzer or the information processing device (specifically, the storage unit), or may be stored in an information storage medium. The information storage medium may be, for example, an SD card, a micro SD card, a CD, a DVD, a flash memory, or a magnetic recording medium.

Note that the present disclosure can also have the following configurations.

[1] A biological sample analyzer including:

an information processing unit configured to perform information processing using signal intensity data on light generated by irradiating a flow channel through which particles are flowing with light, in which

the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

[2] The biological sample analyzer according to [1], in which the information processing unit calculates one or more evaluation values for verifying the status of the device using a data set obtained after the data replacement processing.

[3] The biological sample analyzer according to [2], in which the one or more evaluation values include at least any one of a precision evaluation value of the device, a linearity evaluation value of the device, or a sensitivity evaluation value of the device.

[4] The biological sample analyzer according to any one of [1] to [3], in which signal intensity data of a particle group with the lowest signal intensity among the plurality of types of particle groups is replaced in the data replacement step.

[5] The biological sample analyzer according to any one of [1] to [4], in which

the sample containing the plurality of types of particle groups contains one type of non-fluorescent particle group and one or more types of fluorescent particle groups, and

signal intensity data of the one type of non-fluorescent particle group is replaced in the data replacement step.

[6] The biological sample analyzer according to any one of [1] to [5], configured to perform a trigger slice setting step of setting a trigger slice using the data set.

[7] The biological sample analyzer according to [6], configured to acquire the signal intensity data on light generated by irradiating the flow channel through which no particles are flowing with light using the trigger slice.

[8] The biological sample analyzer according to any one of [1] to [7], configured to perform a background data acquisition step of acquiring the signal intensity data on light generated by irradiating the flow channel through which no particles are flowing with light.

[9] The biological sample analyzer according to [8], in which the acquisition of the signal intensity data in the background data acquisition step is performed in a state where the sample is not flowed into the flow channel, but only a sheath liquid is flowed into the flow channel.

[10] The biological sample analyzer according to [8] or [9], in which the acquisition of the signal intensity data in the background data acquisition step is performed at predetermined time intervals.

[11] The biological sample analyzer according to any one of [8] to [10], in which a number of pieces of the signal intensity data acquired in the background data acquisition step is identical to a number set on the basis of a number of pieces of event data in the data set and a number of peaks formed by the sample.

[12] The biological sample analyzer according to any one of [8] to [11], in which a number of pieces of the signal intensity data acquired in the background acquisition step is identical to a number set on the basis of a number of pieces of the signal intensity data replaced in the data replacement step.

[13] The biological sample analyzer according to any one of [1] to [12], configured to further perform a data separation step of separating the data set into data corresponding to each of the plurality of types of particle groups.

[14] The biological sample analyzer according to [13], configured to perform the data separation through clustering processing in the data separation step.

[15] The biological sample analyzer according to any one of [1] to [14],
the biological sample analyzer configured to replace, in the data replacement step, signal intensity data of a particle group with the lowest fluorescence level among the plurality of types of particle groups with the signal intensity data acquired by irradiating the flow channel through which no particles are flowing with light.

[16] The biological sample analyzer according to any one of [1] to [15], in which

the sample containing the plurality of types of particle groups contains one type of non-fluorescent particle group and one or more types of fluorescent particle groups,
the biological sample analyzer configured to replace, in the data replacement step, signal intensity data of the one type of non-fluorescent particle group with the signal intensity data acquired by irradiating the flow channel through which no particles are flowing with light.

[17] The biological sample analyzer according to any one of [1] to [16], serving as a flow cytometer.

[18] A biological sample analysis system including:

an information processing unit configured to perform information processing using signal intensity data on light generated by irradiating a flow channel through which particles are flowing with light, in which
the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

[19] A method for verifying a status of a biological sample analyzer, the method including:
a data replacement step of causing a sample containing a plurality of types of particle groups having varying fluorescence intensity levels to flow into a flow channel, and replacing signal intensity data on light generated by irradiating, with light, on any one type of particle group in a data set of signal intensity data on light generated by irradiating, with light, particles flowing through the flow channel with signal intensity data acquired by irradiating the flow channel through which no particles are flowing with light.

REFERENCE SIGNS LIST

6100    Biological sample analyzer
6101    Light irradiation unit
6102    Detection unit
6103    Information processing unit

**Claims**

1.  A biological sample analyzer comprising:

an information processing unit configured to perform information processing using signal intensity data on light generated by irradiating a flow channel through which particles are flowing with light, wherein

the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

2. The biological sample analyzer according to claim 1, wherein the information processing unit calculates one or more evaluation values for verifying the status of the device using a data set obtained after the data replacement processing.

3. The biological sample analyzer according to claim 2, wherein the one or more evaluation values include at least any one of a precision evaluation value of the device, a linearity evaluation value of the device, or a sensitivity evaluation value of the device.

4. The biological sample analyzer according to claim 1, wherein signal intensity data of a particle group with the lowest signal intensity among the plurality of types of particle groups is replaced in the data replacement step.

5. The biological sample analyzer according to claim 1, wherein

the sample containing the plurality of types of particle groups contains one type of non-fluorescent particle group and one or more types of fluorescent particle groups, and
signal intensity data of the one type of non-fluorescent particle group is replaced in the data replacement step.

6. The biological sample analyzer according to claim 1, configured to perform a trigger slice setting step of setting a trigger slice using the data set.

7. The biological sample analyzer according to claim 6, configured to acquire the signal intensity data on light generated by irradiating the flow channel through which no particles are flowing with light using the trigger slice.

8. The biological sample analyzer according to claim 1, configured to perform a background data acquisition step of acquiring the signal intensity data on light generated by irradiating the flow channel through which no particles are flowing with light.

9. The biological sample analyzer according to claim 8, wherein the acquisition of the signal intensity data in the background data acquisition step is performed in a state where the sample is not flowed into the flow channel, but only a sheath liquid is flowed into the flow channel.

10. The biological sample analyzer according to claim 8, wherein the acquisition of the signal intensity data in the background data acquisition step is performed at predetermined time intervals.

11. The biological sample analyzer according to claim 8, wherein a number of pieces of the signal intensity data acquired in the background data acquisition step is identical to a number set on a basis of a number of pieces of event data in the data set and a number of peaks formed by the sample.

12. The biological sample analyzer according to claim 8, wherein a number of pieces of the signal intensity data acquired in the background data acquisition step is identical to a number set on a basis of a number of pieces of the signal intensity data replaced in the data replacement step.

13. The biological sample analyzer according to claim 1, configured to further perform a data separation step of separating the data set into data corresponding to each of the plurality of types of particle groups.

14. The biological sample analyzer according to claim 13, configured to perform the data separation through clustering processing in the data separation step.

15. The biological sample analyzer according to claim 1,
the biological sample analyzer configured to replace, in the data replacement step, signal intensity data of a particle group with the lowest fluorescence level among the plurality of types of particle groups with the signal intensity data acquired by irradiating the flow channel through which no particles are flowing with light.

**16.** The biological sample analyzer according to claim 1, wherein

the sample containing the plurality of types of particle groups contains one type of non-fluorescent particle group and one or more types of fluorescent particle groups,

the biological sample analyzer configured to replace, in the data replacement step, signal intensity data of the one type of non-fluorescent particle group with the signal intensity data acquired by irradiating the flow channel through which no particles are flowing with light.

**17.** The biological sample analyzer according to claim 1, serving as a flow cytometer.

**18.** A biological sample analysis system comprising:

an information processing unit configured to perform information processing using signal intensity data on light generated by irradiating a flow channel through which particles are flowing with light, wherein

the information processing unit performs a data replacement step of replacing signal intensity data on light generated by irradiating, with light, any one type of particle group in a data set of signal intensity data on light generated by irradiating, with light, a sample containing a plurality of types of particle groups, the plurality of types of particle groups having varying fluorescence intensity levels, with signal intensity data acquired by irradiating, with light, the flow channel through which no particles are flowing, in verification processing of verifying a status of a device.

**19.** A method for verifying a status of a biological sample analyzer, the method comprising:

a data replacement step of causing a sample containing a plurality of types of particle groups having varying fluorescence intensity levels to flow into a flow channel, and replacing signal intensity data on light generated by irradiating, with light, on any one type of particle group in a data set of signal intensity data on light generated by irradiating, with light, particles flowing through the flow channel with signal intensity data acquired by irradiating the flow channel through which no particles are flowing with light.

# FIG. 1

A

Dim1

B

Dim1

# FIG. 2

S

P

C

6100

6101

LIGHT
IRRADIATION
UNIT

6102

DETECTION
UNIT

6103

INFORMATION
PROCESSING
UNIT

SORTING
UNIT

6104

# FIG. 3

| | |
|---|---|
| DATA SET ACQUISITION STEP | S11 |
| ↓ | |
| TRIGGER SLICE SETTING STEP | S12 |
| ↓ | |
| BACKGROUND DATA ACQUISITION STEP | S13 |
| ↓ | |
| DATA SEPARATION STEP | S14 |
| ↓ | |
| DATA REPLACEMENT STEP | S15 |
| ↓ | |
| EVALUATION VALUE CALCULATION STEP | S16 |

# FIG. 4

# FIG. 5

# FIG. 6

# FIG. 7

|  | REFERENCE SAMPLE (UNSTERILIZED BEADS) | | VERIFICATION SAMPLE (STERILIZED BEADS) | |
|---|---|---|---|---|
|  | MFI (MEASURED) | MEF (KNOWN) | MFI (MEASURED) | MEF (CALCULATED) |
| Dim1 |  | 0 |  | 0 |
| Dim2 | 123,916 | 205 | 86,283 | 143 |
| Dim3 | 277,899 | 470 | 184,447 | 312 |
| Dim4 | 696,304 | 1,211 | 519,588 | 904 |
| Dim5 | 1,656,637 | 2,740 | 1,546,593 | 2,558 |
| Dim6 | 4,661,955 | 7,516 | 4,257,986 | 6,865 |
| Dim7 | 12,986,704 | 20,122 | 12,389,029 | 19,196 |
| Dim8 | 23,508,084 | 35,573 | 22,755,560 | 34,434 |

*FIG. 8*

# FIG. 9

A

| Channel | ⊿Linearity | ⊿MESF | MESF DIFFERENCE RATE | ⊿Q | Q DIFFERENCE RATE | ⊿B | B DIFFERENCE RATE |
|---|---|---|---|---|---|---|---|
| VioBlue | -0.05% | 66.76 | 90.09% | -0.0560 | -14.27% | 153.67 | 83.65% |
| FITC | -0.15% | 448.55 | 130.52% | -0.0004 | -0.94% | -129.99 | -37.69% |
| PE | -0.05% | 110.79 | 113.21% | -0.0117 | -7.14% | 165.24 | 46.40% |
| APC | -0.13% | 94.78 | 108.05% | -0.0320 | -64.65% | 494.30 | 200.00% |

B

| Channel | ⊿Linearity | ⊿MESF | MESF DIFFERENCE RATE | ⊿Q | Q DIFFERENCE RATE | ⊿B | B DIFFERENCE RATE |
|---|---|---|---|---|---|---|---|
| VioBlue | 0.00% | -0.04 | -0.33% | 0.0144 | 3.37% | 7.87 | 7.10% |
| FITC | 0.00% | 0.22 | 0.33% | -0.0008 | -1.88% | 50.18 | 11.54% |
| PE | 0.00% | -0.08 | -0.28% | -0.0044 | -2.63% | 8.01 | 2.89% |
| APC | 0.00% | 0.67 | 2.52% | 0.0011 | 1.67% | 60.38 | 200.00% |

# FIG. 10

# FIG. 11

```
┌─────────────────────┐
│      FLOW STEP      │ ─── S101
└─────────────────────┘
          │
          ▼
┌─────────────────────┐
│ DETERMINATION STEP  │ ─── S102
└─────────────────────┘
          │
          ▼
┌─────────────────────┐
│    RECOVERY STEP    │ ─── S103
└─────────────────────┘
```

# FIG. 12

# FIG. 13

## INTERNATIONAL SEARCH REPORT

| International application No. |
|---|
| **PCT/JP2023/036503** |

**A. CLASSIFICATION OF SUBJECT MATTER**

*G01N 15/1429*(2024.01)i; *G01N 37/00*(2006.01)i; *G01N 21/05*(2006.01)i; *G01N 21/64*(2006.01)i; *G01N 15/14*(2024.01)i
FI:   G01N15/14 B; G01N15/14 C; G01N21/05; G01N21/64 F; G01N37/00 101

According to International Patent Classification (IPC) or to both national classification and IPC

**B. FIELDS SEARCHED**

Minimum documentation searched (classification system followed by classification symbols)

G01N15/14; G01N37/00; G01N21/05; G01N21/64

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2016/185755 A1 (SONY CORP) 24 November 2016 (2016-11-24) | 1-19 |
| A | WO 2019/181205 A1 (SONY CORP) 26 September 2019 (2019-09-26) | 1-19 |
| A | JP 2012-154885 A (OLYMPUS CORP) 16 August 2012 (2012-08-16) | 1-19 |
| A | JP 05-079970 A (TOA MEDICAL ELECTRONICS CO LTD) 30 March 1993 (1993-03-30) | 1-19 |
| A | JP 2015-132622 A (LUMINEX CORP) 23 July 2015 (2015-07-23) | 1-19 |
| A | JP 2021-525359 A (BERKELEY LIGHTS, INC.) 24 September 2021 (2021-09-24) | 1-19 |
| A | WO 2022/066316 A1 (BECTON, DICKINSON AND COMPANY) 31 March 2022 (2022-03-31) | 1-19 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **31 October 2023** | **14 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/036503**

| Patent document cited in search report | | | Publication date (day/month/year) | Patent family member(s) | | | Publication date (day/month/year) |
|---|---|---|---|---|---|---|---|
| WO | 2016/185755 | A1 | 24 November 2016 | JP | 2016-217789 | A | |
| WO | 2019/181205 | A1 | 26 September 2019 | US | 2021/0041342 | A1 | |
| JP | 2012-154885 | A | 16 August 2012 | (Family: none) | | | |
| JP | 05-079970 | A | 30 March 1993 | US | 5548395 | A | |
| | | | | EP | 539022 | A2 | |
| JP | 2015-132622 | A | 23 July 2015 | US | 2012/0002040 | A1 | |
| | | | | WO | 2012/012168 | A2 | |
| | | | | CN | 103003683 | A | |
| | | | | HK | 1212024 | A | |
| JP | 2021-525359 | A | 24 September 2021 | US | 2021/0209752 | A1 | |
| | | | | WO | 2019/232473 | A2 | |
| | | | | CN | 112204380 | A | |
| | | | | KR | 10-2021-0015947 | A | |
| WO | 2022/066316 | A1 | 31 March 2022 | CN | 116348754 | A | |

Form PCT/ISA/210 (patent family annex) (January 2015)

**EP 4 624 896 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016185755 A
- JP 2011232259 A
- JP 2020076736 A